(19) Europäisches Patentamt
European Patent Office
Office européen des brevets

(11) EP 0 976 705 A1

(12) EUROPEAN PATENT APPLICATION

(43) Date of publication:
02.02.2000 Bulletin 2000/05

(51) Int Cl.7: C07C 41/09, C07C 43/205

(21) Application number: 99401871.1

(22) Date of filing: 23.07.1999

(84) Designated Contracting States:
AT BE CH CY DE DK ES FI FR GB GR IE IT LI LU MC NL PT SE
Designated Extension States:
AL LT LV MK RO SI

(30) Priority: 30.07.1998 JP 21500198

(71) Applicant: Ube Industries, Ltd.
Ube-shi, Yamaguchi-prefecture (JP)

(72) Inventors:
- Sugimoto, Tsunemi,
  Ube Lab., Ube Industries, Ltd.
  Ube-shi, Yamaguchi (JP)
- Manabe, Takumi, Ube Lab., Ube Industries, Ltd.
  Ube-shi, Yamaguchi (JP)
- Matsuzaki, Tokuo, Ube Lab., Ube Industries, Ltd.
  Ube-shi, Yamaguchi (JP)

(74) Representative: Portal, Gérard et al
Cabinet Beau de Loménie
158, rue de l'Université
75340 Paris Cédex 07 (FR)

# (54) Method of producing a phenolalkylether compound

(57) A phenol-alkylether compound is produced by a catalytic reaction of a phenol compound of the formula:

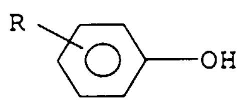

wherein R = H, $C_1$-$C_4$ alkyl, $C_1$-$C_4$ alkoxy, phenyl, benzyl, hydroxyl, nitro or a halogen, with a $C_1$-$C_4$ alkyl alcohol in the presence of a catalyst containing niobic acid which may be anhydrous or hydrated.

EP 0 976 705 A1

## Description

BACKGROUND OF THE INVENTION

(1) Field of the Invention

The present invention relates to a method of producing a phenol-alkylether compound. More particularly, the present invention relates to a method of producing a phenol-alkylether compound by reacting a phenol compound with an alkyl alcohol with a high selectivity for the phenol-alkylether compound. The phenol-alkylether compound refers to an alkyl ether of a phenol compound and is useful as a raw material for producing various medicines, agricultural chemicals (pesticides) and scent materials (perfumes).

(2) Description of the Related Art

In conventional methods of producing a phenol-alkylether compound by reacting a phenol compound with an alkyl alcohol, preferably a lower alkyl alcohol, in the gas phase, Japanese Unexamined Patent Publication No. 52-36,634 discloses a boron phosphate catalyst and Japanese Unexamined Patent Publication No. 53-12,826 discloses a kaolin catalyst. These conventional catalysts are, however, disadvantageous in that the catalytic activity of the catalyst is significantly deteriorated with the lapse of reaction time due to precipitation of a carbon-like substance on the catalyst surface, and thus are quite unsuitable to practical use in industry.

Also, Japanese Unexamined Patent Publication No. 9-235,248 discloses a method of producing a phenolalkyl ether compound by using a oxide catalyst containing an alkali metal. However, this method is disadvantageous in that to produce the phenol-alkylether compound at a satisfactory conversion rate of the phenol compound, the etherification reaction must be carried out at a high reaction temperature.

SUMMARY OF THE INVENTION

An object of the present invention is to provide a method of producing a phenol-alkylether compound from a phenol compound and a lower alkyl alcohol with a high conversion of the phenol compound, with a high selectivity of the phenol-alkylether compound and with a high reaction stability over a long time and with a high industrial applicability of the method.

The above-mentioned object can be attained by the method of the present invention for producing a phenol-alkylether compound, which comprises:

alkyl-etherifying a phenol compound by reacting in gas phase a phenol compound represented by the formula (I):

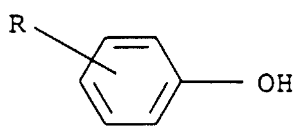

(I)

wherein R represents a member selected from the group consisting of a hydrogen atom, alkyl groups having 1 to 4 carbon atoms, alkoxy groups having 1 to 4 carbon atoms, a phenyl group, a benzyl group, a hydroxyl group, a nitro group and halogen atoms, with an alkyl alcohol having an alkyl group with 1 to 4 carbon atoms, in the presence of a catalyst comprising niobic acid; and

collecting the target phenol-alkylether compound from the resultant reaction mixture.

DESCRIPTION OF THE PREFERRED EMBODIMENTS

The phenol compound from which the target phenol-alkylether compound is produced, is preferably selected from the compounds of the formula (I):

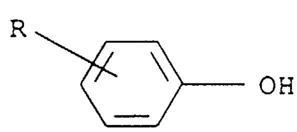

(I)

wherein R represents a member selected from the group consisting of a hydrogen atom, alkyl groups having 1 to 4 carbon atoms, for example, methyl, ethyl, propyl and butyl groups, alkoxyl groups having 1 to 4 carbon atoms, for example, methoxy, ethoxy, propoxy, and butoxy groups, a phenyl group, a benzyl group, a hydroxy group, a nitro group and halogen atoms, for example, fluorine, chlorine, bromine, iodine atoms. For example, the phenol compounds of the formula (I) are selected from phenol, o-cresol, m-cresol, p-cresol, 4-ethylphenol, 4-isopropylphenol, 4-tert-butylphenol, 4-methoxyphenol, 2-ethoxyphenol, 4-phenylphenol, 4-nitrophenyl, 4-benzylphenol and 4-chlorophenol, preferably phenol, o-cresol, m-cresol, p-cresol and catechol.

The alkyl alcohols for producing the phenol-alkylether compounds of the formula (I) preferably include alkyl alcohols in which the alkyl groups have 1 to 4 carbon atoms, for example, methyl alcohol, ethyl alcohol, n-propyl alcohol and n-butyl alcohol, more preferably methyl alcohol and ethyl alcohol.

In the alkyletherification reaction of the method of present invention, the alkyl alcohol is preferably present in an amount of 1 to 100 moles, more preferably 1 to 20 moles, per mole of the starting phenol compound. When the amount of the alkyl alcohol is less than 1 mole per mole of the phenol compound, the conversion of the phenol compound to the target phenol alkylether compound may be low and an economical disadvantage may occur, and when the amount of the alkyl alcohol is more than 100 moles per mole of the phenol compound, the amount of the non-reacted alkyl alcohol is large and thus a cost for recovering the non-reacted alkyl alcohol may be high and an economical disadvantage may occur.

In the phenol-alkylether compound-producing reaction of the present invention, the starting phenol compound and alkyl alcohol are vaporized by using a vaporizer and then resultant vapor mixture is fed into a reactor. Optionally, an inert gas, for example, nitrogen, helium or argon gas is fed together with the vapor mixture into the reactor. In the feeding procedure, the weight hour space velocity (WHSV) of the vapor mixture is preferably 0.01 to 100 $h^{-1}$, more preferably 0.1 to 10 $h^{-1}$.

In the method of the present invention, the reaction of the phenol compound of the formula (I) with the alkyl alcohol is carried out in gas phase in the presence of a catalyst comprising niobic acid. The niobic acid is of the formula:

$$Nb_2O_5 \cdot xH_2O$$

wherein x represent an integer of 0 or 1 or more.

The niobic acid for the method of the present invention is preferably one heat-treated at a temperature of 100 to 500°C for the time of, for example, 0.5 to 5 hours, in air or an inert gas atmosphere.

In the method of the present invention, the alkyletherification reaction is effected by, for example, feeding a mixed vapor of the starting phenol compound and alkyl alcohol into a reactor packed with the niobic acid catalyst under the ambient pressure or a pressure higher than the ambient pressure, preferably at a reaction temperature of 200 to 400°C, more preferably 200 to 300°C and for the time of 0.01 to 100 seconds.

The resultant reaction mixture in the state of a vapor is delivered from the reactor and is subjected to an isolation and refining procedure of the target phenol-alkylether compound.

The target phenol-alkylether compound is collected from the resultant reaction mixture by the isolation procedure, for example, a distillation in which the target phenol-alkylether compound is collected as a liquid fraction.

## EXAMPLES

The present invention will be further illustrated by the following examples.

### Example 1

A heat-resistant glass tubular reactor having a length of 50 cm and an inner diameter of 2.6 cm was packed with 18 ml (16g) of niobic acid ground into a grain size of 1 to 2 mm (available from CBMM INTERNATIONAL LIMITADA CO), and was filled with nitrogen gas. The niobic acid grains in the reactor were heat-treated at a temperature of 300°C for one hour in the nitrogen gas atmosphere.

Separately, a mixture of methyl alcohol with phenol in a mixing molar ratio of 9:1 was fed under the ambient pressure into a vaporizer at a weight hour space velocity (WHSV) of 0.8 $h^{-1}$ together with nitrogen gas fed at a flow rate of 10 ml/min, and vaporized in the vaporizer.

The resultant vapor mixture was successively fed into the reactor packed with the heat-treated niobic acid catalyst at the same WHSV as mentioned above, and was subjected to an etherification reaction at a temperature of 250°C.

The resultant reaction mixture vapor was delivered from the reactor, and cooled with ice, to collect a reaction mixture in the state of a liquid. The liquid fraction collected 2 hours after the start of feeding of the vapor mixture into

the reactor was subjected to a gas-chromatographic analysis. It was confirmed that the conversion of phenol was 49% and the selectivity of anisole was 86%.

Example 2

The same procedures as in Example 1 were carried out except that the reaction temperature was changed from 250°C to 220°C.

The liquid fraction collected 2 hours after the start of the feeding of the vapor mixture into the reactor was subjected to a gas chromatographic analysis. It was confirmed that the conversion of phenol was 23% and the selectivity of anisole was 89%.

Example 3

The same procedures as in Example 1 were carried out except that the WHSV of the vapor mixture was changed to 1.5 $h^{-1}$. The resultant liquid fractions collected at the stages of the times shown in Table 1 (9 to 400 hours) after the start of the feeding of the vapor mixture into the reactor were subjected to the gas chromatographic analysis. The analysis results are shown in Table 1.

Table 1

| Time after the start of reaction (h) | Conversion of phenol (%) | Selectivity of anisole (%) |
|---|---|---|
| 9 | 27 | 86 |
| 20 | 26 | 86 |
| 40 | 26 | 86 |
| 60 | 25 | 86 |
| 80 | 25 | 86 |
| 100 | 25 | 86 |
| 150 | 23 | 85 |
| 200 | 24 | 85 |
| 250 | 23 | 85 |
| 300 | 21 | 85 |
| 350 | 22 | 85 |
| 400 | 22 | 85 |

Example 4

The same procedures as in Example 1 were carried out except that the heat treatment of the niobic acid was carried out in the air atmosphere at a temperature of 500°C for 3 hours.

The liquid fraction collected 2 hours after the start of the feeding of the vapor mixture into the reactor was subjected to the gas chromatographic analysis. It was confirmed that the conversion of phenol of 33% and the selectivity of anisole was 86%.

Example 5

The same procedures as in Example 1 were carried out except that phenol was replaced by catechol in a molar ratio of methyl alcohol to catechol of 3.4:1, and the reaction temperature was changed to 230°C. The reaction mixture was collected 2 hours after the start of feeding the vapor mixture and subjected to the gas chromatographic analysis. It was confirmed that the conversion of catechol was 31%, the selectivity of the resultant guaiacol was 91% and the selectivity of the resultant veratrol was 1%.

The method of the present invention enables a phenol-alkylether compound to be produced from a phenol compound and a lower alkyl alcohol with a high conversion of the starting phenol compound, with a high selectivity of the target phenol-alkylether compound and with a high stability over a long time of reaction, and thus is an industrially

useful method of producing a phenol-alkylether compound.

## Claims

1. A method of producing a phenol-alkylether compound comprising:

   alkyl-etherifying a phenol compound by reacting in gas phase a phenol compound represented by the formula (I):

   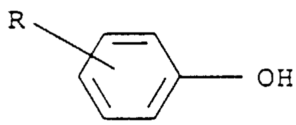

   (I)

   wherein R represents a member selected from the group consisting of a hydrogen atom, alkyl groups having 1 to 4 carbon atoms, alkoxy groups having 1 to 4 carbon atoms, a phenyl group, a benzyl group, a hydroxyl group, a nitro group and halogen atoms, with an alkyl alcohol having an alkyl group with 1 to 4 carbon atoms, in the presence of a catalyst comprising niobic acid; and
   collecting the target phenol-alkylether compound from the resultant reaction mixture.

2. The method of producing a phenol-alkylether compound as claimed in claim 1, wherein the niobic acid is one heat-treated at a temperature of 100 to 500°C.

3. The method of producing a phenol-alkylether compound as claimed in claim 1 or 2, wherein the alkyl alcohol is present in an amount of 1 to 100 moles per mole of the phenol compound.

4. The method of producing a phenol-alkylether compound as claimed in any one of claims 1 to 3, wherein the phenol compound and the alkyl alcohol are fed into the reaction thereof at a weight hour space velocity of 0.01 to 100 $h^{-1}$.

5. The method for producing a phenol-alkylether compound as claimed in any of claims 1 to 4, wherein the reaction of the phenol compound with the alkyl alcohol is carried our at a temperature of 200 to 400°C.

6. The method of producing a phenol-alkylether compound as claimed in any of claims 1 to 5, wherein the target phenol-alkylether compound is collected by distillation.

European Patent Office

# EUROPEAN SEARCH REPORT

Application Number
EP 99 40 1871

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | PATENT ABSTRACTS OF JAPAN<br>vol. 96, no. 4, 30 April 1996 (1996-04-30)<br>& JP 07 324050 A (SUMITOMO CHEM),<br>12 December 1995 (1995-12-12)<br>* abstract *<br>--- | 1-6 | C07C41/09<br>C07C43/205 |
| A | EP 0 781 751 A (NIPPON SHOKUBAI)<br>2 July 1997 (1997-07-02)<br>* claims; example 32 *<br>----- | 1 | |

TECHNICAL FIELDS SEARCHED (Int.Cl.7)
C07C

The present search report has been drawn up for all claims

| Place of search | Date of completion of the search | Examiner |
|---|---|---|
| THE HAGUE | 26 October 1999 | Wright, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 99 40 1871

This annex lists the patent family members relating to the patent documents cited in the above-mentioned European search report.
The members are as contained in the European Patent Office EDP file on
The European Patent Office is in no way liable for these particulars which are merely given for the purpose of information.

26-10-1999

| Patent document cited in search report | Publication date | Patent family member(s) | Publication date |
|---|---|---|---|
| JP 07324050 A | 12-12-1995 | NONE | |
| EP 781751 A | 02-07-1997 | CN 1157283 A | 20-08-1997 |
| | | JP 2865165 B | 08-03-1999 |
| | | JP 9235248 A | 09-09-1997 |
| | | US 5817886 A | 06-10-1998 |

EPO FORM P0459

For more details about this annex : see Official Journal of the European Patent Office, No. 12/82